# EUROPEAN PATENT APPLICATION

(11) **EP 0 835 928 A1**
(43) Date of publication of application: **15.04.1998**
(21) Application number: 96116337.5
(22) Date of filing: 11.10.1996
(51) Int. Cl.: C12N 1/21, C12N 15/31, C12N 15/74, A61K 39/02, A61K 39/112, C12Q 1/68

(54) **Helicobacter pylori live vaccine**

(71) Applicant: Max-Planck-Gesellschaft zur Förderung der Wissenschaften e.V. Berlin, 80539 München (DE)
(72) Inventor: Meyer, Thomas, Prof.Dr., 72076 Tübingen (DE); Gòmez, Oscar, Dr., 72076 Tübingen (DE); Yan, Zhengxin, Dr., 72076 Tübingen (DE); Haas, Rainer, Dr., 72076 Tübingen (DE); Lucas, Bernadette, Dr., 72076 Tübingen (DE)
(74) Representative: Weiss, Wolfgang, Dipl.-Chem. Dr.

(57) **Abstract**

The present invention relates to novel recombinant live vaccines, which provide protective immunity against an infection by Helicobacter pylori and a method of screening H. pylori antigens for optimized vaccines.

SEQUENCE LISTING

(1) GENERAL INFORMATION:

(i) APPLICANT:
(A) NAME: Max-Planck-Gesellschaft zur Foerderung der
Wissenschaften e.V. Berlin
(B) STREET: Hofgartenstr. 2
(C) CITY: Muenchen
(E) COUNTRY: Germany
(F) POSTAL CODE (ZIP): 80539

(ii) TITLE OF INVENTION: Helicobacter pylori live vaccine

(iii) NUMBER OF SEQUENCES: 4

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:
(A) LENGTH: 1557 base pairs
(B) TYPE: nucleic acid
(C) STRANDEDNESS: both
(D) TOPOLOGY: linear

(ii) MOLECULE TYPE: DNA (genomic)

(vi) ORIGINAL SOURCE:
(A) ORGANISM: Helicobacter pylori

(vii) IMMEDIATE SOURCE:
(B) CLONE: alpB

(ix) FEATURE:
(A) NAME/KEY: CDS
(B) LOCATION:1..1554

(xi) SEQUENCE DESCRIPTION: SEQ ID NO: 1:

ATG ACA CAA TCT CAA AAA GTA AGA TTC TTA GCC CCT TTA AGC CTA GCG 48
Met Thr Gln Ser Gln Lys Val Arg Phe Leu Ala Pro Leu Ser Leu Ala
1               5                   10                  15

TTA AGC TTG AGC TTC AAT CCA GTG GGC GCT GAA GAA GAT GGG GGC TTT 96
Leu Ser Leu Ser Phe Asn Pro Val Gly Ala Glu Glu Asp Gly Gly Phe
            20                  25                  30

ATG ACC TTT GGG TAT GAA TTA GGT CAG GTG GTC CAA CAA GTG AAA AAC 144
Met Thr Phe Gly Tyr Glu Leu Gly Gln Val Val Gln Gln Val Lys Asn
        35                  40                  45

CCG GGT AAA ATC AAA GCC GAA GAA TTA GCC GGC TTG TTA AAC TCT ACC 192
Pro Gly Lys Ile Lys Ala Glu Glu Leu Ala Gly Leu Leu Asn Ser Thr
    50                  55                  60

ACA ACA AAC AAC ACC AAT ATC AAT ATT GCA GGC ACA GGA GGC AAT GTC 240

Thr Thr Asn Asn Thr Asn Ile Asn Ile Ala Gly Thr Gly Gly Asn Val
65                  70                  75                  80

GCC GGG ACT TTG GGC AAC CTT TTT ATG AAC CAA TTA GGC AAT TTG ATT     288
Ala Gly Thr Leu Gly Asn Leu Phe Met Asn Gln Leu Gly Asn Leu Ile
                85                  90                  95

GAT TTG TAT CCC ACT TTG AAC ACT AGT AAT ATC ACA CAA TGT GGC ACT     336
Asp Leu Tyr Pro Thr Leu Asn Thr Ser Asn Ile Thr Gln Cys Gly Thr
            100                 105                 110

ACT AAT AGT GGT AGT AGT AGT AGT GGT GGT GGT GCG GCC ACA GCC GCT     384
Thr Asn Ser Gly Ser Ser Ser Ser Gly Gly Gly Ala Ala Thr Ala Ala
        115                 120                 125

GCT ACT ACT AGC AAT AAG CCT TGT TTC CAA GGT AAC CTG GAT CTT TAT     432
Ala Thr Thr Ser Asn Lys Pro Cys Phe Gln Gly Asn Leu Asp Leu Tyr
    130                 135                 140

AGA AAA ATG GTT GAC TCT ATC AAA ACT TTG AGT CAA AAC ATC AGC AAG     480
Arg Lys Met Val Asp Ser Ile Lys Thr Leu Ser Gln Asn Ile Ser Lys
145                 150                 155                 160

AAT ATC TTT CAA GGC AAC AAC AAC ACC ACG AGC CAA AAT CTC TCC AAC     528
Asn Ile Phe Gln Gly Asn Asn Asn Thr Thr Ser Gln Asn Leu Ser Asn
                165                 170                 175

CAG CTC AGT GAG CTT AAC ACC GCT AGC GTT TAT TTG ACT TAC ATG AAC     576
Gln Leu Ser Glu Leu Asn Thr Ala Ser Val Tyr Leu Thr Tyr Met Asn
            180                 185                 190

TCG TTC TTA AAC GCC AAT AAC CAA GCG GGT GGG ATT TTT CAA AAC AAC     624
Ser Phe Leu Asn Ala Asn Asn Gln Ala Gly Gly Ile Phe Gln Asn Asn
        195                 200                 205

ACT AAT CAA GCT TAT GGA AAT GGG GTT ACC GCT CAA CAA ATC GCT TAT     672
Thr Asn Gln Ala Tyr Gly Asn Gly Val Thr Ala Gln Gln Ile Ala Tyr
    210                 215                 220

ATC CTA AAG CAA GCT TCA ATC ACT ATG GGG CCA AGC GGT GAT AGC GGT     720
Ile Leu Lys Gln Ala Ser Ile Thr Met Gly Pro Ser Gly Asp Ser Gly
225                 230                 235                 240

GCT GCC GCA GCG TTT TTG GAT GCC GCT TTA GCG CAA CAT GTT TTC AAC     768
Ala Ala Ala Ala Phe Leu Asp Ala Ala Leu Ala Gln His Val Phe Asn
                245                 250                 255

TCC GCT AAC GCC GGG AAC GAT TTG AGC GCT AAG GAA TTC ACT AGC TTG     816
Ser Ala Asn Ala Gly Asn Asp Leu Ser Ala Lys Glu Phe Thr Ser Leu
            260                 265                 270

GTG CAA AAT ATC GTC AAT AAT TCT CAA AAC GCT TTA ACG CTA GCC AAC     864
Val Gln Asn Ile Val Asn Asn Ser Gln Asn Ala Leu Thr Leu Ala Asn
        275                 280                 285

AAC GCT AAC ATC AGC AAT TCA ACA GGC TAT CAA GTG AGC TAT GGC GGG     912
Asn Ala Asn Ile Ser Asn Ser Thr Gly Tyr Gln Val Ser Tyr Gly Gly
    290                 295                 300

AAT ATT GAT CAA GCG CGA TCT ACC CAA CTA TTA AAC AAC ACC ACA AAC     960
Asn Ile Asp Gln Ala Arg Ser Thr Gln Leu Leu Asn Asn Thr Thr Asn
305                 310                 315                 320

ACT TTG GCT AAA GTT AGC GCT TTG AAT AAC GAG CTT AAA GCT AAC CCA     1008

Thr Leu Ala Lys Val Ser Ala Leu Asn Asn Glu Leu Lys Ala Asn Pro
                325                 330                 335

TGG CTT GGG AAT TTT GCC GCC GGT AAC AGC TCT CAA GTG AAT GCG TTT 1056
Trp Leu Gly Asn Phe Ala Ala Gly Asn Ser Ser Gln Val Asn Ala Phe
            340                 345                 350

AAC GGG TTT ATC ACT AAA ATC GGT TAC AAG CAA TTC TTT GGG GAA AAC 1104
Asn Gly Phe Ile Thr Lys Ile Gly Tyr Lys Gln Phe Phe Gly Glu Asn
        355                 360                 365

AAG AAT GTG GGC TTA CGC TAC TAC GGC TTC TTC AGC TAT AAC GGC GCG 1152
Lys Asn Val Gly Leu Arg Tyr Tyr Gly Phe Phe Ser Tyr Asn Gly Ala
    370                 375                 380

GGC GTG GGT AAT GGC CCT ACT TAC AAT CAA GTC AAT TTG CTC ACT TAT 1200
Gly Val Gly Asn Gly Pro Thr Tyr Asn Gln Val Asn Leu Leu Thr Tyr
385                 390                 395                 400

GGG GTG GGG ACT GAT GTG CTT TAC AAT GTG TTT AGC CGC TCT TTT GGT 1248
Gly Val Gly Thr Asp Val Leu Tyr Asn Val Phe Ser Arg Ser Phe Gly
                405                 410                 415

AGT AGG AGT CTT AAT GCG GGC TTC TTT GGG GGG ATC CAA CTC GCA GGG 1296
Ser Arg Ser Leu Asn Ala Gly Phe Phe Gly Gly Ile Gln Leu Ala Gly
            420                 425                 430

GAT ACT TAC ATC AGC ACG CTA AGA AAC AGC TCT CAG CTT GCG AGC AGA 1344
Asp Thr Tyr Ile Ser Thr Leu Arg Asn Ser Ser Gln Leu Ala Ser Arg
        435                 440                 445

CCT ACA GCG ACG AAA TTC CAA TTC TTG TTT GAT GTG GGC TTA CGC ATG 1392
Pro Thr Ala Thr Lys Phe Gln Phe Leu Phe Asp Val Gly Leu Arg Met
    450                 455                 460

AAC TTT GGT ATC TTG AAA AAA GAC TTG AAA AGC CAT AAC CAG CAT TCT 1440
Asn Phe Gly Ile Leu Lys Lys Asp Leu Lys Ser His Asn Gln His Ser
465                 470                 475                 480

ATA GAA ATC GGT GTG CAA ATC CCT ACG ATT TAC AAC ACT TAC TAT AAA 1488
Ile Glu Ile Gly Val Gln Ile Pro Thr Ile Tyr Asn Thr Tyr Tyr Lys
                485                 490                 495

GCT GGC GGT GCT GAA GTG AAA TAC TTC CGC CCT TAT AGC GTG TAT TGG 1536
Ala Gly Gly Ala Glu Val Lys Tyr Phe Arg Pro Tyr Ser Val Tyr Trp
            500                 505                 510

GTC TAT GGC TAC GCC TTC TAA 1557
Val Tyr Gly Tyr Ala Phe
        515

(2) INFORMATION FOR SEQ ID NO: 2:

(i) SEQUENCE CHARACTERISTICS:
(A) LENGTH: 518 amino acids
(B) TYPE: amino acid
(D) TOPOLOGY: linear

(ii) MOLECULE TYPE: protein
(xi) SEQUENCE DESCRIPTION: SEQ ID NO: 2:

Met Thr Gln Ser Gln Lys Val Arg Phe Leu Ala Pro Leu Ser Leu Ala
1               5                   10                  15

Leu Ser Leu Ser Phe Asn Pro Val Gly Ala Glu Glu Asp Gly Gly Phe
            20                  25                  30

Met Thr Phe Gly Tyr Glu Leu Gly Gln Val Val Gln Gln Val Lys Asn
        35                  40                  45

Pro Gly Lys Ile Lys Ala Glu Glu Leu Ala Gly Leu Leu Asn Ser Thr
    50                  55                  60

Thr Thr Asn Asn Thr Asn Ile Asn Ile Ala Gly Thr Gly Gly Asn Val
65                  70                  75                  80

Ala Gly Thr Leu Gly Asn Leu Phe Met Asn Gln Leu Gly Asn Leu Ile
                85                  90                  95

Asp Leu Tyr Pro Thr Leu Asn Thr Ser Asn Ile Thr Gln Cys Gly Thr
            100                 105                 110

Thr Asn Ser Gly Ser Ser Ser Ser Gly Gly Gly Ala Ala Thr Ala Ala
        115                 120                 125

Ala Thr Thr Ser Asn Lys Pro Cys Phe Gln Gly Asn Leu Asp Leu Tyr
    130                 135                 140

Arg Lys Met Val Asp Ser Ile Lys Thr Leu Ser Gln Asn Ile Ser Lys
145                 150                 155                 160

Asn Ile Phe Gln Gly Asn Asn Asn Thr Thr Ser Gln Asn Leu Ser Asn
                165                 170                 175

Gln Leu Ser Glu Leu Asn Thr Ala Ser Val Tyr Leu Thr Tyr Met Asn
            180                 185                 190

Ser Phe Leu Asn Ala Asn Asn Gln Ala Gly Gly Ile Phe Gln Asn Asn
        195                 200                 205

Thr Asn Gln Ala Tyr Gly Asn Gly Val Thr Ala Gln Gln Ile Ala Tyr
    210                 215                 220

Ile Leu Lys Gln Ala Ser Ile Thr Met Gly Pro Ser Gly Asp Ser Gly
225                 230                 235                 240

Ala Ala Ala Ala Phe Leu Asp Ala Ala Leu Ala Gln His Val Phe Asn
                245                 250                 255

Ser Ala Asn Ala Gly Asn Asp Leu Ser Ala Lys Glu Phe Thr Ser Leu
            260                 265                 270

Val Gln Asn Ile Val Asn Asn Ser Gln Asn Ala Leu Thr Leu Ala Asn
        275                 280                 285

Asn Ala Asn Ile Ser Asn Ser Thr Gly Tyr Gln Val Ser Tyr Gly Gly
    290                 295                 300

Asn Ile Asp Gln Ala Arg Ser Thr Gln Leu Leu Asn Asn Thr Thr Asn
305                 310                 315                 320

Thr Leu Ala Lys Val Ser Ala Leu Asn Asn Glu Leu Lys Ala Asn Pro
                325                 330                 335

Trp Leu Gly Asn Phe Ala Ala Gly Asn Ser Ser Gln Val Asn Ala Phe
            340                 345                 350

Asn Gly Phe Ile Thr Lys Ile Gly Tyr Lys Gln Phe Phe Gly Glu Asn
        355                 360                 365

Lys Asn Val Gly Leu Arg Tyr Tyr Gly Phe Phe Ser Tyr Asn Gly Ala
    370                 375                 380

Gly Val Gly Asn Gly Pro Thr Tyr Asn Gln Val Asn Leu Leu Thr Tyr
385                 390                 395                 400

Gly Val Gly Thr Asp Val Leu Tyr Asn Val Phe Ser Arg Ser Phe Gly
                405                 410                 415

Ser Arg Ser Leu Asn Ala Gly Phe Phe Gly Gly Ile Gln Leu Ala Gly
            420                 425                 430

Asp Thr Tyr Ile Ser Thr Leu Arg Asn Ser Ser Gln Leu Ala Ser Arg
        435                 440                 445

Pro Thr Ala Thr Lys Phe Gln Phe Leu Phe Asp Val Gly Leu Arg Met
    450                 455                 460

Asn Phe Gly Ile Leu Lys Lys Asp Leu Lys Ser His Asn Gln His Ser
465                 470                 475                 480

Ile Glu Ile Gly Val Gln Ile Pro Thr Ile Tyr Asn Thr Tyr Tyr Lys
                485                 490                 495

Ala Gly Gly Ala Glu Val Lys Tyr Phe Arg Pro Tyr Ser Val Tyr Trp
            500                 505                 510

Val Tyr Gly Tyr Ala Phe
        515

(2) INFORMATION FOR SEQ ID NO: 3:

    (i) SEQUENCE CHARACTERISTICS:
        (A) LENGTH: 1557 base pairs
        (B) TYPE: nucleic acid
        (C) STRANDEDNESS: both
        (D) TOPOLOGY: linear

    (ii) MOLECULE TYPE: DNA (genomic)

    (vi) ORIGINAL SOURCE:
        (A) ORGANISM: Helicobacter pylori

    (vii) IMMEDIATE SOURCE:
        (B) CLONE: alpA

    (ix) FEATURE:
        (A) NAME/KEY: CDS
        (B) LOCATION:1..1554

    (xi) SEQUENCE DESCRIPTION: SEQ ID NO: 3:

ATG ATA AAA AAG AAT AGA ACG CTG TTT CTT AGT CTA GCC CTT TGC GCT      48
Met Ile Lys Lys Asn Arg Thr Leu Phe Leu Ser Leu Ala Leu Cys Ala
    520                 525                 530

AGC ATA AGT TAT GCC GAA GAT GAT GGA GGG TTT TTC ACC GTC GGT TAT      96
Ser Ile Ser Tyr Ala Glu Asp Asp Gly Gly Phe Phe Thr Val Gly Tyr
535                 540                 545                 550

CAG CTC GGG CAA GTC ATG CAA GAT GTC CAA AAC CCA GGC GGC GCT AAA 144
Gln Leu Gly Gln Val Met Gln Asp Val Gln Asn Pro Gly Gly Ala Lys
555 560 565

AGC GAC GAA CTC GCC AGA GAG CTT AAC GCT GAT GTA ACG AAC AAC ATT 192
Ser Asp Glu Leu Ala Arg Glu Leu Asn Ala Asp Val Thr Asn Asn Ile
570 575 580

TTA AAC AAC AAC ACC GGA GGC AAC ATC GCA GGG GCG TTG AGT AAC GCT 240
Leu Asn Asn Asn Thr Gly Gly Asn Ile Ala Gly Ala Leu Ser Asn Ala
585 590 595

TTC TCC CAA TAC CTT TAT TCG CTT TTA GGG GCT TAC CCC ACA AAA CTC 288
Phe Ser Gln Tyr Leu Tyr Ser Leu Leu Gly Ala Tyr Pro Thr Lys Leu
600 605 610

AAT GGT AGC GAT GTG TCT GCG AAC GCT CTT TTA AGT GGT GCG GTA GGC 336
Asn Gly Ser Asp Val Ser Ala Asn Ala Leu Leu Ser Gly Ala Val Gly
615 620 625 630

TCT GGG ACT TGT GCG GCT GCA GGG ACG GCT GGT GGC ACT TCT CTT AAC 384
Ser Gly Thr Cys Ala Ala Ala Gly Thr Ala Gly Gly Thr Ser Leu Asn
635 640 645

ACT CAA AGC ACT TGC ACC GTT GCG GGC TAT TAC TGG CTC CCT AGC TTG 432
Thr Gln Ser Thr Cys Thr Val Ala Gly Tyr Tyr Trp Leu Pro Ser Leu
650 655 660

ACT GAC AGG ATT TTA AGC ACG ATC GGC AGC CAG ACT AAC TAC GGC ACG 480
Thr Asp Arg Ile Leu Ser Thr Ile Gly Ser Gln Thr Asn Tyr Gly Thr
665 670 675

AAC ACC AAT TTC CCC AAC ATG CAA CAA CAG CTC ACC TAC TTG AAT GCG 528
Asn Thr Asn Phe Pro Asn Met Gln Gln Gln Leu Thr Tyr Leu Asn Ala
680 685 690

GGG AAT GTG TTT TTT AAT GCG ATG AAT AAG GCT TTA GAG AAT AAG AAT 576
Gly Asn Val Phe Phe Asn Ala Met Asn Lys Ala Leu Glu Asn Lys Asn
695 700 705 710

GGA ACT AGT AGT GCT AGT GGA ACT AGT GGT GCG ACT GGT TCA GAT GGT 624
Gly Thr Ser Ser Ala Ser Gly Thr Ser Gly Ala Thr Gly Ser Asp Gly
715 720 725

CAA ACT TAC TCC ACA CAA GCT ATC CAA TAC CTT CAA GGC CAA CAA AAT 672
Gln Thr Tyr Ser Thr Gln Ala Ile Gln Tyr Leu Gln Gly Gln Gln Asn
730 735 740

ATC TTA AAT AAC GCA GCG AAC TTG CTC AAG CAA GAT GAA TTG CTC TTA 720
Ile Leu Asn Asn Ala Ala Asn Leu Leu Lys Gln Asp Glu Leu Leu Leu
745 750 755

GAA GCT TTC AAC TCT GCC GTA GCC GCC AAC ATT GGG AAT AAG GAA TTC 768
Glu Ala Phe Asn Ser Ala Val Ala Ala Asn Ile Gly Asn Lys Glu Phe
760 765 770

AAT TCA GCC GCT TTT ACA GGT TTG GTG CAA GGC ATT ATT GAT CAA TCT 816
Asn Ser Ala Ala Phe Thr Gly Leu Val Gln Gly Ile Ile Asp Gln Ser
775 780 785 790

CAA GCG GTT TAT AAC GAG CTC ACT AAA AAC ACC ATT AGC GGG AGT GCG 864
Gln Ala Val Tyr Asn Glu Leu Thr Lys Asn Thr Ile Ser Gly Ser Ala
795 800 805

GTT ATT AGC GCT GGG ATA AAC TCC AAC CAA GCT AAC GCT GTG CAA GGG     912
Val Ile Ser Ala Gly Ile Asn Ser Asn Gln Ala Asn Ala Val Gln Gly
            810                 815                 820

CGC GCT AGT CAG CTC CCT AAC GCT CTT TAT AAC GCG CAA GTA ACT TTG     960
Arg Ala Ser Gln Leu Pro Asn Ala Leu Tyr Asn Ala Gln Val Thr Leu
        825                 830                 835

GAT AAA ATC AAT GCG CTC AAT AAT CAA GTG AGA AGC ATG CCT TAC TTG     1008
Asp Lys Ile Asn Ala Leu Asn Asn Gln Val Arg Ser Met Pro Tyr Leu
    840                 845                 850

CCC CAA TTC AGA GCC GGG AAC AGC CGT TCA ACG AAT ATT TTA AAC GGG     1056
Pro Gln Phe Arg Ala Gly Asn Ser Arg Ser Thr Asn Ile Leu Asn Gly
855                 860                 865                 870

TTT TAC ACC AAA ATA GGC TAT AAG CAA TTC TTC GGG AAG AAA AGG AAT     1104
Phe Tyr Thr Lys Ile Gly Tyr Lys Gln Phe Phe Gly Lys Lys Arg Asn
                875                 880                 885

ATC GGT TTG CGC TAT TAT GGT TTC TTT TCT TAT AAC GGA GCG AGC GTG     1152
Ile Gly Leu Arg Tyr Tyr Gly Phe Phe Ser Tyr Asn Gly Ala Ser Val
            890                 895                 900

GGC TTT AGA TCC ACT CAA AAT AAT GTA GGG TTA TAC ACT TAT GGG GTG     1200
Gly Phe Arg Ser Thr Gln Asn Asn Val Gly Leu Tyr Thr Tyr Gly Val
        905                 910                 915

GGG ACT GAT GTG TTG TAT AAC ATC TTT AGC CGC TCC TAT CAA AAC CGC     1248
Gly Thr Asp Val Leu Tyr Asn Ile Phe Ser Arg Ser Tyr Gln Asn Arg
    920                 925                 930

TCT GTG GAT ATG GGC TTT TTT AGC GGT ATC CAA TTA GCC GGT GAG ACC     1296
Ser Val Asp Met Gly Phe Phe Ser Gly Ile Gln Leu Ala Gly Glu Thr
935                 940                 945                 950

TTC CAA TCC ACG CTC AGA GAT GAC CCC AAT GTG AAA TTG CAT GGG AAA     1344
Phe Gln Ser Thr Leu Arg Asp Asp Pro Asn Val Lys Leu His Gly Lys
                955                 960                 965

ATC AAT AAC ACG CAC TTC CAG TTC CTC TTT GAC TTC GGT ATG AGG ATG     1392
Ile Asn Asn Thr His Phe Gln Phe Leu Phe Asp Phe Gly Met Arg Met
            970                 975                 980

AAC TTC GGT AAG TTG GAC GGG AAA TCC AAC CGC CAC AAC CAG CAC ACG     1440
Asn Phe Gly Lys Leu Asp Gly Lys Ser Asn Arg His Asn Gln His Thr
        985                 990                 995

GTG GAA TTT GGC GTA GTG GTG CCT ACG ATT TAT AAC ACT TAT TAC AAA     1488
Val Glu Phe Gly Val Val Val Pro Thr Ile Tyr Asn Thr Tyr Tyr Lys
    1000                1005                1010

TCA GCA GGG ACT ACC GTG AAG TAT TTC CGT CCT TAT AGC GTT TAT TGG     1536
Ser Ala Gly Thr Thr Val Lys Tyr Phe Arg Pro Tyr Ser Val Tyr Trp
1015                1020                1025                1030

TCT TAT GGG TAT TCA TTC TAA                                         1557
Ser Tyr Gly Tyr Ser Phe
                1035

(2) INFORMATION FOR SEQ ID NO: 4:

    (i) SEQUENCE CHARACTERISTICS:

(A) LENGTH: 518 amino acids
(B) TYPE: amino acid
(D) TOPOLOGY: linear

(ii) MOLECULE TYPE: protein
(xi) SEQUENCE DESCRIPTION: SEQ ID NO: 4:

Met Ile Lys Lys Asn Arg Thr Leu Phe Leu Ser Leu Ala Leu Cys Ala
1               5                   10                  15

Ser Ile Ser Tyr Ala Glu Asp Asp Gly Gly Phe Phe Thr Val Gly Tyr
            20                  25                  30

Gln Leu Gly Gln Val Met Gln Asp Val Gln Asn Pro Gly Gly Ala Lys
        35                  40                  45

Ser Asp Glu Leu Ala Arg Glu Leu Asn Ala Asp Val Thr Asn Asn Ile
    50                  55                  60

Leu Asn Asn Asn Thr Gly Gly Asn Ile Ala Gly Ala Leu Ser Asn Ala
65                  70                  75                  80

Phe Ser Gln Tyr Leu Tyr Ser Leu Leu Gly Ala Tyr Pro Thr Lys Leu
                85                  90                  95

Asn Gly Ser Asp Val Ser Ala Asn Ala Leu Leu Ser Gly Ala Val Gly
            100                 105                 110

Ser Gly Thr Cys Ala Ala Ala Gly Thr Ala Gly Gly Thr Ser Leu Asn
        115                 120                 125

Thr Gln Ser Thr Cys Thr Val Ala Gly Tyr Tyr Trp Leu Pro Ser Leu
    130                 135                 140

Thr Asp Arg Ile Leu Ser Thr Ile Gly Ser Gln Thr Asn Tyr Gly Thr
145                 150                 155                 160

Asn Thr Asn Phe Pro Asn Met Gln Gln Gln Leu Thr Tyr Leu Asn Ala
                165                 170                 175

Gly Asn Val Phe Phe Asn Ala Met Asn Lys Ala Leu Glu Asn Lys Asn
            180                 185                 190

Gly Thr Ser Ser Ala Ser Gly Thr Ser Gly Ala Thr Gly Ser Asp Gly
        195                 200                 205

Gln Thr Tyr Ser Thr Gln Ala Ile Gln Tyr Leu Gln Gly Gln Gln Asn
    210                 215                 220

Ile Leu Asn Asn Ala Ala Asn Leu Leu Lys Gln Asp Glu Leu Leu Leu
225                 230                 235                 240

Glu Ala Phe Asn Ser Ala Val Ala Ala Asn Ile Gly Asn Lys Glu Phe
                245                 250                 255

Asn Ser Ala Ala Phe Thr Gly Leu Val Gln Gly Ile Ile Asp Gln Ser
            260                 265                 270

Gln Ala Val Tyr Asn Glu Leu Thr Lys Asn Thr Ile Ser Gly Ser Ala
        275                 280                 285

Val Ile Ser Ala Gly Ile Asn Ser Asn Gln Ala Asn Ala Val Gln Gly
    290                 295                 300

Arg Ala Ser Gln Leu Pro Asn Ala Leu Tyr Asn Ala Gln Val Thr Leu
305                 310                 315                 320

Asp Lys Ile Asn Ala Leu Asn Asn Gln Val Arg Ser Met Pro Tyr Leu
                325                 330                 335

Pro Gln Phe Arg Ala Gly Asn Ser Arg Ser Thr Asn Ile Leu Asn Gly
            340                 345                 350

Phe Tyr Thr Lys Ile Gly Tyr Lys Gln Phe Phe Gly Lys Lys Arg Asn
        355                 360                 365

Ile Gly Leu Arg Tyr Tyr Gly Phe Phe Ser Tyr Asn Gly Ala Ser Val
    370                 375                 380

Gly Phe Arg Ser Thr Gln Asn Asn Val Gly Leu Tyr Thr Tyr Gly Val
385                 390                 395                 400

Gly Thr Asp Val Leu Tyr Asn Ile Phe Ser Arg Ser Tyr Gln Asn Arg
                405                 410                 415

Ser Val Asp Met Gly Phe Phe Ser Gly Ile Gln Leu Ala Gly Glu Thr
            420                 425                 430

Phe Gln Ser Thr Leu Arg Asp Asp Pro Asn Val Lys Leu His Gly Lys
        435                 440                 445

Ile Asn Asn Thr His Phe Gln Phe Leu Phe Asp Phe Gly Met Arg Met
    450                 455                 460

Asn Phe Gly Lys Leu Asp Gly Lys Ser Asn Arg His Asn Gln His Thr
465                 470                 475                 480

Val Glu Phe Gly Val Val Val Pro Thr Ile Tyr Asn Thr Tyr Tyr Lys
                485                 490                 495

Ser Ala Gly Thr Thr Val Lys Tyr Phe Arg Pro Tyr Ser Val Tyr Trp
            500                 505                 510

Ser Tyr Gly Tyr Ser Phe
        515

## Description

The present invention relates to novel recombinant live vaccines, which provide protective immunity against an infection by Helicobacter pylori and a method of screening H. pylori antigens for optimized vaccines.

Helicobacter is a gram-negative bacterial pathogen associated with the development of gastritis, pepic ulceration and gastric carcinoma. Several Helicobacter species colonize the stomach, most notably H. pylori, H. heilmanii and H. felis. Although H. pylori is the species most commonly associated with human infection, H. heilmanii and H. felis also have been found to infect humans. High H. pylori infection rates are observed in third world countries, as well as in industrialized countries. Among all the virulence factors described in H. pylori, urease is known to be essential for colonisation of gnobiotic pigs and nude mice. Urease is an enzyme composed of two structural subunits (UreA and UreB). Previous studies have indicated that oral immunization using recombinant UreB plus cholera toxin were able to protect mice from gastric colonisation with H. felis and H. pylori (Michetti et al., Gastroenterology 107 (1994), 1002-1011). By oral administration of recombinant UreB antigens, however, in several cases only an incomplete protection can be obtained. Other H. pylori antigens shown to give partial protection are the 87 kD vacuolar cytotoxin VacA (Cover and Blaser, J. Biol. Chem. 267 (1992), 10570; Marchetti et al., Science 267 (1995), 1655) and the 13 and 58 kD heat shock proteins HspA and HspB (Ferrero et al., Proc. Natl. Acad. Sci. USA 92 (1995), 6499).

Attenuated pathogens, e.g. bacteria, such as Salmonella, are known to be efficient live vaccines. The first indications of the efficacy of attenuated Salmonella as good vaccine in humans came from studies using a chemically mutagenized Salmonella typhi Ty21a strain (Germanier and Furer, J. Infect. Dis. 141 (1975), 553-558), tested successfully in adult volunteers (Gilman et al., J. Infect. Dis. 136 (1977), 717-723) and later on in children in a large field trial in Egypt (Whadan et al., J. Infect. Dis. 145 (1982), 292-295). The orally administered Ty21a vaccine was able to protect 96% of the Egyptian children vaccinated during three years of surveillance. Since that time new attenuated Salomonella live vector vaccines have developed (Hone et al., Vaccine 9 (1991), 810-816), in which well defined mutations incorporated into the chromosome gave rise to non-virulent strains able to induce strong immune responses after oral administration (Tacket et al., Vaccine 10 (1992), 443-446 and Tacket et al., Infect. Immun. 60 (1992), 536-541). Other advantages of the live attenuated Salmonella vaccine include its safety, easy administration, long-time protection and no adverse reactions in comparison with the former inactivated wholesale typhoid vaccines (Levine et al., Typhoid Fever Vaccines. In: Plotkin S.A., Mortimer E.A. Jr. (eds.) Vaccines. Philadelphia: WB Saunders (1988), 333-361).

Mutants of S. typhimurium have been extensively used to deliver antigens because of the possibility to use mice as an animal model, which is believed to mimick S. typhi infections in humans. The attenuation of S. typhimurium most commonly used consists in site directed mutagenesis of genes affecting the synthesis of aromatic amino acids. Such strains, designated aro mutants, have a negligible pathogenicity, as demonstrated in animal models and human trials using these constructs (Hoiseth and Stocker, Nature 291 (1981), 238-239; Tacket et al. (1992), Supra). Advantage has been taken from the potent immunogenicity of live Salmonella vaccine to deliver heterologous antigens. Expression of specific antigens in attenuated Salmonella has conferred murine protection against several bacterial pathogens. The use of recombinant live vaccines, which are capable of expressing Helicobacter antigens and protecting the vaccinated animals, has not yet been described.

The use of attenuated live vaccines for the treatment of a Helicobacter infection has also not been rendered obvious. The reason therefor being that in the course of the Helicobacter infection a strong immune response against the pathogen per se is induced, which, however, does not lead to a protective immunity. Thus, it was highly surprising that a protective immune response is achieved when using recombinant attenuated bacterial cells as antigen carriers, which are capable of expressing a DNA molecule encoding a Helicobacter antigen. Apparently, recombinant attenuated bacterial cells expressing a Helicobacter antigen are capable of creating a qualitatively different immune response against the heterologous Helicobacter antigen than Helicobacter itself does against its own homologous antigen. Surprisingly, a non-protective immune response is thus transformed into an immune response protecting against Helicobacter infections. This unexpected observation renders it possible to use recombinant attenuated pathogens, e.g. bacterial cells, particularly Salmonella, as carriers for the screening of protective antigens, to apply the protective antigens identified in this manner in any vaccine against Helicobacter infections, and to use recombinant attenuated bacteria as carriers of protective antigens for the immunization against Helicobacter infections in humans and other mammals.

Thus, a subject matter of the present invention is a recombinant attenuated pathogen, which comprises at least one heterologous nucleic acid molecule encoding a Helicobacter antigen, wherein said pathogen is capable to express said nucleic acid molecule or capable to cause the expression of said nucleic acid in a target cell. Preferably the nucleic acid molecule is a DNA molecule.

The attenuated pathogen is a microorganism strain which is able to cause infection and preferably effective immunological protection against the actual pathogen but is no longer pathogenic per se. The attenuated pathogen can be a bacterium, a virus, a fungus or a parasite. Preferably it is a bacterium, e.g. Salmonella, such as S. typhimurium or S. typhi, Vibrio cholerae (Mekalanos et al., Nature 306 (1983), 551-557), Shigella Species such as S. flexneri (Sizemore et al., Science 270 (1995), 299-302; Mounier et al., EMBO J. 11 (1992), 1991-1999), Listeria such as L. monocytogenes (Milon and Cossart, Trends in Microbiology 3 (1995), 451-453), Escherichia coli, Streptococcus, such as S. gordonii (Medaglini et al., Proc. Natl. Acad. Sci. USA 92 (1995) 6868-6872) or Mycobacterium, such as Bacille Calmette Guerin (Flynn, Cell. Mol. Biol. 40 Suppl. 1 (1994), 31-36). More preferably the pathogen is an attenuated enterobacterium such as Vibrio cholerae, Shigella flexneri, Escherichia coli or Salmonella. Most preferably the attenuated pathogen is a Salmonella cell, e.g. a Salmonella aro mutant cell. The attenuated pathogen, however, can be a virus, e.g. an attenuated vaccinia virus, adenovirus or pox virus.

The nucleic acid molecule which is inserted into the pathogen codes for a Helicobacter antigen, preferably a H. felis, H. heilmanii or H. pylori antigen, more preferably a H. pylori antigen. The Helicobacter antigen can be a native Helicobacter polypeptide, an immunologically reactive fragment thereof, or an immunologically reactive variant of a native polypeptide or of a fragment thereof. Further, the Helicobacter antigen can be a protective carbohydrate or a peptide mimotope simulating the three-dimensional structure of a native Helicobacter antigen. Peptide mimotopes can be obtained from peptide libraries presented on the surface of bacterial cells (cf. PCT/EP96/01130). Of course, the transformed cell can also contain several DNA molecules coding for different Helicobacter antigens.

Attenuated bacteria can be used to transcribe and translate said nucleic acid molecule directly in the bacterial cell or to deliver said nucleic acid molecule to the infected target cell, such that the DNA molecule is transcribed and/or translated by the eukaryotic target cell machinery. This indirect bacterial vaccination procedure, termed here as genetic vaccination, has been successfully used with Shigella as a carrier (Sizemore, D. R., Branstrom, A. A. & Sadoff, J. C. (1995) Attenuated Shigella as a DNA delivery vehicle for DNA-mediated immunization. Science 270:299-302).

In a preferred embodiment of the present invention the Helicobacter antigen is urease, a urease subunit or an immunologically reactive variant or fragment thereof or a peptide mimotope thereof. In a further preferred embodiment of the present invention the Helicobacter antigen is a secretory polypeptide from Helicobacter, an immunologically reactive variant or fragment thereof or a peptide mimotope thereof. A process for identifying Helicobacter genes coding for such secretory polypeptides, and particularly for adhesins, has been disclosed in the international patent application PCT/EP96/02544, which is incorporated herein by reference. This process comprises
a) preparing a gene bank of H. pylori DNA in a host organism containing an inducible transposon coupled to a marker of secretory activity,
b) inducing the insertion of the transposon into the H. pylori DNA and
c) conducting a selection for clones containing a secretory gene by means of the marker, and optionally further
d) conducting a retransformation of H. pylori by means of the DNA of clones containing genes having secretory activity, wherein isogenic H. pylori mutant strains are produced by means of integrating the DNA into the chromosome, and
e) conducting a selection detecting adherence-deficient H. pylori mutant strains.

Suitable examples of antigens obtainable by the above process are selected from the group consisting of the antigens AlpA, AlpB, immunologically reactive variants or fragments thereof or peptide mimotopes thereof. The nucleic and amino acid sequences of the antigens AlpA and AlpB have been disclosed in the international patent applications PCT/EP96/02545 and PCT/EP96/04124, which are incorporated herein by reference. Further, the nucleic and amino acid sequences of AlpB are shown in SEQ ID NO. 1 and 2, and the nucleic and amino acid sequences of AlpA in SEQ ID NO. 3 and 4.

It is also conceivable, however, that an intracellular antigen is used which can be presented on the surface, e.g. by autolytic release, and confers immunological protection.

The presentation of the Helicobacter antigens in the recombinant pathogen according to the invention can be accomplished in different ways. The antigen or the antigens can be synthesized in a constitutive, inducible or phase variable manner in the recombinant pathogen. Concerning the constitutive or inducible synthesis of the Helicobacter antigens known expression systems can be referred to, as have been described by Sambrook et al., Molecular Cloning, A Laboratory Manual (1989), Cold Spring Harbor Laboratory Press.

Particularly preferred the antigens are presented in a phase variable expression system. Such a phase variable expression system for the production and presentation of foreign antigens in hybrid live vaccines is disclosed in EP-B-0 565 548, which is herein incorporated by reference. In such a phase variable expression system the nucleic acid molecule encoding the Helicobacter antigen is under control of an expression signal, which is substantially inactive in the pathogen, and which is capable of being activated by a spontaneous reorganization caused by a nucleic acid, e.g. DNA reorganization mechanism in the pathogen, e.g. a specific DNA inversion process, a specific DNA deletion process, a specific DNA replication process or a specific slipped-strand-mispairing mechanism.

A recombinant cell having a phase variable expression system is capable of forming two subpopulations A and B, wherein the division into said subpopulations occurs by spontaneous reorganization in the recombinant nucleic acid, wherein said sub-population A is capable of infection and immunologically active per se, while subpopulation B, which is regenerated from subpopulation A, produces at least one heterologous Helicobacter antigen and acts immunologically with respect to said additional antigen.

The activation of the expression signal encoding the Helicobacter antigen can be directly accomplished by nucleic acid reorganization or, alternatively, indirectly accomplished by activation of a gene encoding a protein which controls the expression of the gene encoding the Helicobacter antigen. The indirect activation represents a system which allows the production of the Helicobacter antigen via a cascade system, which can be realized e.g. in that the gene directly controlled by DNA reorganization codes for an RNA polymerase which is specific for the promoter preceding the Helicobacter gene, or a gene regulator which in another specific manner induces the expression of the Helicobacter gene. In an especially preferred embodiment of the present invention the expression signal for the gene encoding the Helicobacter antigen is a bacteriophage promoter, e.g. a T3, T7 or SP6 promoter, and the activation of the expression signal is caused by a nucleic acid reorganization resulting in the production of a corresponding bacteriophage RNA polymerase in the pathogen.

The phase variable expression system can be adjusted to provide a preselected expression level of the Helicobacter antigen. This can be accomplished e.g. by modifying the nucleotide sequence of the expression signal, which is activated by the nucleic acid reorganization mechanism, and/or by inserting further genetic regulation elements.

The Helicobacter antigens can be produced in an intracellular, as well as in an extracellular manner in the pathogen according to the invention. For instance, autotransporter systems such as the IgA-protease system (cf. for instance EP-A-0 254 090) or the E. coli AIDA-1 adhesin system (Benz et al., Mol. Microbiol. 6 (1992), 1539) are suited as extracellular secretory system. Other suitable outer membrane transporter systems are the RTX-toxin transporters, e.g. the E. coli hemolysin transport system (Hess et al., Proc. Natl. Acad. Sci. USA 93 (1996), 11458-11463).

The pathogen according to the invention can contain a second heterologous nucleic acid, e.g. DNA molecule, which codes for an immunomodulatory polypeptide influencing the immune response quantitatively or qualitatively, apart from the nucleic acid molecule encoding the Helicobacter antigen. Examples of such immunomodulatory polypeptides are immune-stimulating peptides, cytokines like IL-2, IL-6 or IL-12, chemokines, toxins, such as cholera toxin B or adhesins.

The present invention also refers to a pharmaceutical composition comprising as an active agent a recombinant attenuated pathogen as described above, optionally together with pharmaceutically acceptable diluents, carriers and adjuvants. Preferably, the composition is a living vaccine. The vaccination routes depend upon the choice of the vaccination vector. The administration may be achieved in a single dose or repeated at intervals. The appropriate dosage depends on various parameters such as the vaccinal vector itself, or the route of administration. Administration to a mucosal surface (e.g. ocular, intranasal, oral, gastric, intestinal, rectal, vaginal or urinary tract) or via the parenteral route (e.g. subcutaneous, intradermal, intramuscular, intravenous or intraperitoneal) might be chosen. A method for the preparation of the living vaccine comprises formulating the attenuated pathogen in a pharmaceutically effective amount with pharmaceutically acceptable diluents, carriers and/or adjuvants.

Further, the present invention refers to a method for preparing a recombinant attenuated pathogen as defined above, comprising the steps of a) inserting a nucleic acid molecule encoding a Helicobacter antigen into an attenuated pathogen, wherein the recombinant pathogen, e.g. a transformed bacterial cell, is obtained, which is capable of expressing said nucleic acid molecule or is capable to cause expression of said nucleic acid molecule in a target cell and b) cultivating said recombinant attenuated pathogen under suitable conditions. If the pathogen is a bacterial cell, the nucleic acid molecule encoding the Helicobacter antigen can be located on an extrachromosomal plasmid. It is, however, also possible to insert the nucleic acid molecule into the chromosome of the pathogen.

Furthermore, the present invention refers to a method for identifying Helicobacter antigens which raise a protective immune response in a mammalian host, comprising the steps of:
a) providing an expression gene bank of Helicobacter in an attenuated pathogen and b) screening the clones of the gene bank for the ability to confer a protective immunity against a Helicobacter infection in a mammalian host. Preferably, this identification process takes place in a phase variable expression system, rendering possible a stable expression of all of the Helicobacter antigens. Recombinant clones can then be applied as "pools" for the oral immunization of test animals, such as mice. The potential of these clones as protective antigen is then determined via a challenge infection with Helicobacter, e.g. a mouse-adapted H. pylori strain. Thus, there is a possibility of directly selecting optimized H. pylori vaccine antigens.

The invention will be further illustrated by the following figures and sequence listings.

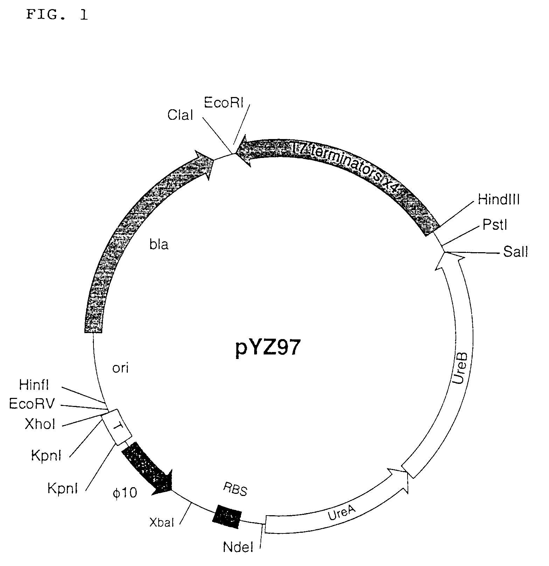

- Fig. 1:: shows a schematic illustration of the urease expression vector pYZ97, whereon the genes coding for the urease subunits UreA and UreB are located under transcriptional control of the T7 promoter φ10. There is a ribosomal binding site (RBS) between the T7 promoter and the urease genes. Further, the plasmid exhibits an origin of replication (ori), a β-lactamase resistance gene (bla) and 4 T7 terminators in series.
Apart from the expression by the T7 promoter, a constitutive low level expression of the urease A and B subunits can also be brought about via a cryptic promoter, which is located upstream from the T7 promoter, on the plasmid pYZ97.

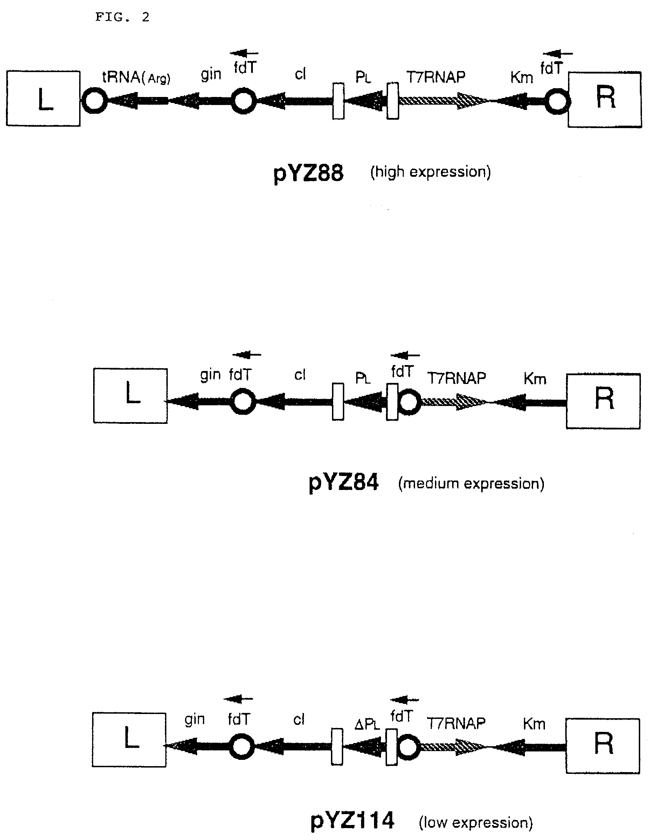

- Fig. 2:: shows a schematic illustration of the T7 RNA polymerase (T7RNAP) expression cassettes pYZ88, pYZ84 and pYZ114, which can be integrated into the chromosomes of bacteria.
In the high-expression cassette pYZ88 the lambda PL promoter is located in inverse orientation, upstream from the T7RNAP gene. A gene for the temperature-sensitive repressor cI 857 (cI) is under control of this promoter. A terminator of the bacteriophage fd (fdT) is situated upstream from the cI gene. The gin gene (Mertens, EMBO J. 3 (1984), 2415-2421) codes for a control enzyme of a DNA reorganization mechanism. A DNA sequence coding for the tRNA Arg is located downstream from the gin gene.
In phase A the PL promoter responsible for the expression of the T7RNAP gene is directed in the direction of the cI857 gene and the gin gene. The consequence of this is that an active repressor is formed at the permissive temperature of 28°C and reduces the transcription from the PL promoter. At a higher temperature the transcription of the PL promoter is increased, since the repressor is inactivated at least partially under such external influences. The temperature-dependent increase in the transcription also causes a corresponding increase in the expression of the following gin gene, which as a control enzyme catalyses the inversion of the PL promoter and the transition in phase B, in which the T7RNAP gene is expressed.
In the high-expression system pYZ88 a further fdT transcription terminator is located between a kanamycin-resistance gene (km) and the promoter of this gene. In this manner, the synthesis of an anti-sense RNA, inversely orientated to the T7RNAP gene, which normally contributes to the reduction of the T7RNAP expression, is reduced. This results in a high expression of the T7RNAP.
In the medium-expression system pYZ84 a transcription terminator (fdT) is located between the PL promoter and the start of the T7RNAP gene. In this manner the expression of the T7RNAP mRNA is reduced. Additionally, the anti-sense RNA affects the T7RNAP translation. Therefore, only a medium expression occurs.
In the low-expression system pYZ114 a deletion of 100 bp in PL is additionally introduced (Δ PL). In this manner the activity of the PL promoter is reduced to a high extent, which leads to a lower T7RNAP expression and thus to a reduction of the UreA/B gene expression. In this construct the effect of the cryptic promoter on pYZ97 is already observed.

SEQ ID NO. 1 and 2 show the nucleotide sequence of the adhesin gene AlpB from H. pylori and the amino acid sequence of the polypeptide coded therefrom.
SEQ ID N0. 3 and 4 show the nucleotide sequence of the adhesin gene AlpA from H. pylori and the amino acid sequence of the protein coded therefrom.

### Experimental part

### Materials and Methods

Bacterial strains: S. typhimurium SL3261 live vector vaccine strain was used as a recipient for the recombinant H. pylori urease plasmid constructs. S. typhimurium SL3261 is an aroA transposon mutant derived from S. typhimurium SL1344 wild type strain. S. typhimurium SL3261 is a non-virulent strain that gives protection to mice against infection with wild type S. typhimurium after oral administration (Hoiseth and Stocker (1981) Supra). S. typhimurium SL3261 and derivatives thereof, which contain the urease expression plasmid pYZ97 (extrachromosomal) and the T7RNAP expression cassettes pYZ88, pYZ84 or pYZ114, respectively (integrated into the chromosome) are indicated in table 1. Luria broth or agar was used for bacterial growth at 28°C. H. pylori wild type strain grown at 37°C on serum plates was used for the challenge experiments.

Immunization of mice: Four weeks Balb/c mice purchased from Interfauna (Tuttlingen, Germany) were adapted two weeks in an animal facility before being used for experimentation. 150 µl of blood was taken retroorbitally from all mice to obtain preimmune serum. Retroorbital bleedings were repeated from all immunized mice 1 week and 3 weeks after immunization.

Eight groups of 5 mice including controls were used in this study (table 2). Group A, the naive control group, was not immunized with Salmonella neither challenged with wild type H. pylori. The rest of the groups were all orally immunized. Group B, a negative control group, did not receive Salmonella and was challenged with H. pylori. Mice from groups C to G were immunized with Salmonella vaccine strains and challenged with H. pylori. The last group H received recombinant urease B in combination with cholera toxin and was also challenged.

Prior to immunizations mice were left overnight without solid food and 4 hours without water. 100 µl of 3% sodium bicarbonate were given orally using a stainless steel catheter tube to neutralize the stomach pH. Then mice from group B received 100 µl PBS and mice from groups C to G received 1.0 x 10¹⁰ CFU of Salmonella in a 100 µl volume. Mice from group H received four times 100 µl of a mixture of recombinant H. pylori UreaseB plus cholera toxin, one dose every week. After every immunization water and food were returned to the mice.

H. pylori challenge: Four weeks after the first oral immunization mice from groups B to H were challenged with H.pylori. Mice were left overnight without solid food and without water 4 hours prior to the challenge. 100 µl of 3% sodium bicarbonate were given orally to the mice using a stainless steel catheter tube, followed by an oral dose of 5.0 x 10⁹ CFU/ml of Helicobacter pylori. Water and food were returned to the mice after the challenge.

Collection of blood and tissues from mice: Twelve weeks after the first immunization the mice were left overnight without food and subsequently sacrificed for analysis of protection and immune response. The mice were anaesthetized with Metoxyfluorane for terminal cardiac bleeding and prior to sacrifice by cervical dislocation. Under aseptic conditions, spleen and stomach were carefully removed from each mouse and placed on ice in separate sterile containers until further processing. Large and small intestine were obtained for further isolation of the intestinal fluid.

Processing of stomach and measurement of urease activity: The degree of H. pylori colonisation in the mouse stomach was measured by the presence of active urease in the tissue. The Jatrox-test (Röhm-Pharma GmbH, Weiterstadt, Germany) was used according to the suppliers' directions. Stomach mucosa was exposed and washed with PBS, half of the antral portion of the stomach was immediately placed inside an Eppendorf tube containing the substrate for measurement of urease activity. Absorbance at 550 nm was measured after tubes were incubated for 4 hours at room temperature. The rest of the stomach tissue was stored at -20°C for further treatments. The urease activity values obtained from the stomach of naive mice, which did not undergo immunization or challenge, were used to create a base line to indicate the absence of H. pylori infection and therefore protection.

**Table 1**

| **UreA and UreB expressing S. typhimurium vaccine strains** | | |
|---|---|---|
| Strains | Urease Expression | Source |
| S. typhimurium SL3261 | Negative | Hoiseth and Stocker |
| S. typhimurium SL3262 pYZ97 | Constitutive Low | this study |
| S. typhimurium SL3261::pYZ88pYZ97 | High T7-induced expression | this study |
| S. typhimurium SL3261::pYZ84pYZ97 | Medium T7-induced expression | this study |
| S. typhimurium SL3261::pYZ114pYZ97 | Low T7-induced expression | this study |

**Table 2**

| **Mice groups used for immunization** | | |
|---|---|---|
| Group | Immunogen | No. of oral immunizations |
| A | None | 0 |
| B | PBS oral immunization | 1 |
| C | S. typhimurium S3261 | 1 |
| D | S. typhimurium S3261 pYZ97 | 1 |
| E | S. typhimurium S3261::pYZ88pYZ97 | 1 |
| F | S. typhimurium S3261::pYZ84pYZ97 | 1 |
| G | S. typhimurium S3261::pYZ114pYZ97 | 1 |
| H | Urease B plus cholera toxin | 4 |

### Results:

In the control mice (groups B and C) 100% infection with H. pylori was observed. In the mice immunized with recombinant attenuated pathogens (groups D, E, F, G) between 0% and 60% infection (100% to 40% protection) was observed. Immuno-protection did not correlate with humoral anti-UreA and UreB response, suggesting that, in addition to humoral immunity, cellular immunity is critical for protection against H. pylori infection. The results indicate that oral immunization of mice with UreA and UreB delivered by S. typhimurium attenuated strain is effective to induce high levels of protection against H. pylori colonisation.

In the mice immunized with recombinant urease B plus cholera toxin considerably higher levels of urease activity were observed under said experimental conditions than when administering the recombinant attenuated pathogens according to the invention.

The results of the urease test have been illustrated in table 3.

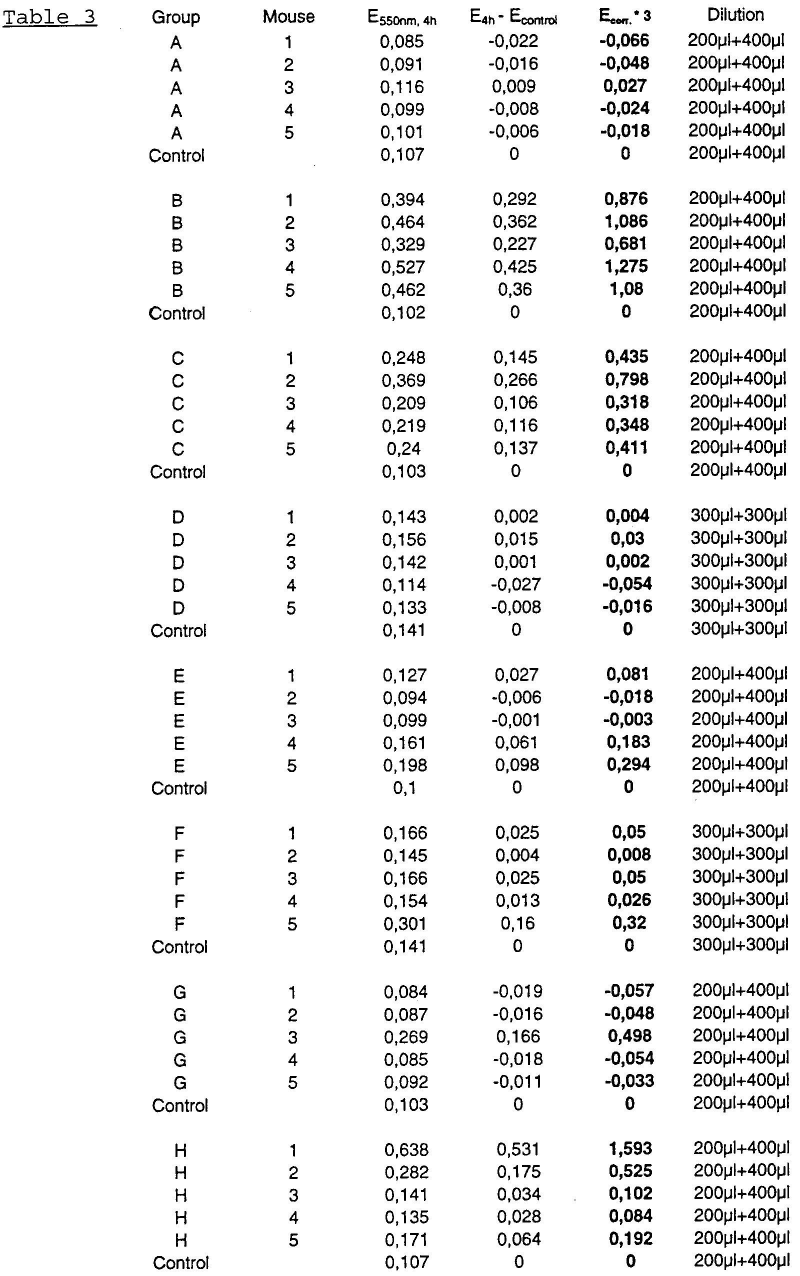

## Claims

1. A recombinant attenuated microbial pathogen, which comprises at least one heterologous nucleic acid molecule encoding a Helicobacter antigen, wherein said pathogen is capable to express said nucleic acid molecule or capable to cause the expression of said nucleic acid molecule in a target cell.

2. The pathogen according to claim 1, which is an enterobacterial cell, especially a Salmonella cell.

3. The pathogen according to claim 1 or 2, which is a Salmonella aro mutant cell.

4. The pathogen according to any of claims 1-3,
wherein the Helicobacter antigen is urease, a urease subunit, an immunologically reactive fragment thereof, or a peptide mimotope thereof.

5. The pathogen according to any one of claims 1-3,
wherein the Helicobacter antigen is a secretory polypeptide from Helicobacter, an immunologically reactive fragment thereof, or a peptide mimotope thereof.

6. The pathogen according to any one of claims 1-3 and 5, wherein the Helicobacter antigen is selected from the group consisting of the antigens AlpA, AlpB, immunologically reactive fragments thereof, or a peptide mimotope thereof.

7. The pathogen according to any one of claims 1-6, wherein said nucleic acid molecule encoding a Helicobacter antigen is capable to be expressed phase variably.

8. The pathogen according to claim 7,
wherein said nucleic acid molecule encoding the Helicobacter antigen is under control of an expression signal which is substantially inactive in the pathogen and which is capable to be activated by a nucleic acid reorganization caused by a nucleic acid reorganization mechanism in the pathogen.

9. The pathogen according to claim 8,
wherein the expression signal is a bacteriophage promoter, and the activation is caused by a DNA reorganization resulting in the production of a corresponding bacteriophage RNA polymerase in the pathogen.

10. The pathogen according to any one of claims 1-9, further comprising at least one second nucleic acid molecule encoding an immunomodulatory polypeptide, wherein said pathogen is capable to express said second nucleic acid molecule.

11. Pharmaceutical composition comprising as an active agent a recombinant attenuated pathogen according to any one of claims 1-10, optionally together with pharmaceutically acceptable diluents, carriers and adjuvants.

12. Composition according to claim 11, which is a living vaccine, which is suitable for administration to a mucosal surface or via the parenteral route.

13. A method for the preparation of a living vaccine comprising formulating an attenuated pathogen according to any one of claims 1-10 in a pharmaceutically effective amount with pharmaceutically acceptable diluents, carriers and/or adjuvants.

14. A method for preparing a recombinant attenuated pathogen according to any one of claims 1-10, comprising the steps:
a) inserting a nucleic acid molecule encoding a Helicobacter antigen into an attenuated pathogen, wherein a recombinant attenuated pathogen is obtained, which is capable of expressing said nucleic acid molecule or is capable to cause expression of said nucleic acid molecule in a target cell, and
b) cultivating said recombinant attenuated pathogen under suitable conditions.

15. The method according to claim 15,
wherein said nucleic acid molecule encoding a Helicobacter antigen is located on an extrachromosomal plasmid.

16. A method for identifying Helicobacter antigens, which raise a protective immune response in a mammalian host, comprising the steps of:
a) providing an expression gene bank of Helicobacter in an attenuated pathogen and
b) screening the clones of the gene bank for their ability to confer protective immunity against a Helicobacter infection in a mammalian host.
